(19) Europäisches Patentamt
European Patent Office
Office européen des brevets

(11) Publication number: 0 143 461
B1

# (12) EUROPEAN PATENT SPECIFICATION

(45) Date of publication of patent specification: 06.09.89

(51) Int. Cl.[4]: C 07 D 277/36, A 61 K 31/425, C 07 D 417/06

(21) Application number: 84114373.8

(22) Date of filing: 28.11.84

## (54) Rhodanine derivative, process for preparing the same and pharmaceutical composition containing the same.

(30) Priority: 29.11.83 JP 226488/83
24.08.84 JP 177210/84

(43) Date of publication of application:
05.06.85 Bulletin 85/23

(45) Publication of the grant of the patent:
06.09.89 Bulletin 89/36

(84) Designated Contracting States:
CH DE FR GB IT LI NL SE

(56) References cited:
EP-A-0 045 165
EP-A-0 047 109
FR-A-2 148 647

(73) Proprietor: Kaken Pharmaceutical Co., Ltd.
No. 28-8, Honkomagome 2-chome Bunkyo-ku
Tokyo (JP)

(72) Inventor: Ohishi, Yoshitaki
102-10, Minamiura
Ogura-c-o Uji-shi Kyoto-fu (JP)
Inventor: Nagahara, Michiko
591, Oaza-Nagahara
Yasu-cho Yasu-gun Shiga-ken (JP)
Inventor: Takehisa, Yoshitaka
13, Kaginote-cho
Omihachiman-shi Shiga-ken (JP)
Inventor: Yajima, Motoyuki
31-9-406, Ohira 2-chome
Otsu-shi Shiga-ken (JP)
Inventor: Kurokawa, Shigeki
28-8, Seta 3-chome
Otsu-shi Shiga-ken (JP)
Inventor: Kajikawa, Norio
4-16, Nishino-otezaki-cho Yamashina-ku
Kyoto-shi Kyoto-fu (JP)
Inventor: Itoh, Akira
7-21, Nishigori 1-chome
Otsu-shi Shiga-ken (JP)
Inventor: Nogimori, Katsumi
7-6, Oe 7-chome
Otsu-shi Shiga-ken (JP)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 143 461 B1

Courier Press, Leamington Spa, England.

(74) Representative: **Türk, Gille, Hrabal et al**
**Bruckner Strasse 20**
**D-4000 Düsseldorf 13 (DE)**

**Description**

The present invention relates to novel rhodanine derivatives, processes for preparing the same and a pharmaceutical composition containing the same as a therapeutic agent for diabetic complications.

In recent years, westernization of eating habits has resulted in a marked increase in the number of diabetic patients and measures for the treatment thereof are urgently needed.

As therapeutic agents for diabetes, insulin and blood sugar lowering agents have so far been used widely. However, diabetes is not a mere disorder of sugar metabolism but a disease also involving a variety of complications and therefore the therapeutic effects of the above-mentioned agents alone are not enough for the treatment of diabetes.

Among main complications, cerebral and coronary vascular disturbances account for about 50% of causes of deaths resulting from diabetes [Y. Goto et al., Sogo Rinsho, *22*, 779 to 785 (1973)].

Blood platelets play an important role in the development of such vascular disturbances. Thus, in a diabetic condition, platelets are in the state of hyperfunction, causing thrombosis and at the same time arteriosclerosis [H. Heath et al., Diabetologia, *7*, 308 to 315 (1971)]. Therefore, platelet aggregation inhibitors are useful in the treatment of vascular disturbances such as mentioned above.

On the other hand, diseases of the eye, such as retinopathy and cataract, are also important diabetic complications and from the primary cause of blindness of the aged. In the development of such diseases, not only disturbance of retinal and other blood vessels, namely microangiopathy, is an important pathogenic factor, but also a certain kind of sugar metabolism disorder is concerned [K. H. Gabbay, Adv. Metab. Disord., *2*(2), 424 1973)]. Thus, in the diabetic condition, polyols such as sorbitol are accumulated to an extraordinary extent, causing osmotic pressure increase and water retention, which lead to ocular tissue disturbance. Therefore, inhibition of aldose reductase which is essential to polyol synthesis can possibly be effective in the prevention and treatment of the above-mentioned diseases of the eye [R. G. Judzewitsch et al., New Eng. J. Med., *308*, 119 to 125 (1983); J. H. Kinoshita et al., Metabolism, *28* (1), 462, to 469 (1979)].

It is an object of the present invention to provide a novel compound useful as a therapeutic agent for diabetic complications including perceptual disorder, autonomic disturbance, diabetic nephropathy, and ocular diseases such as retinopathy and cataract.

This and other objects of the present invention will become apparent from the description hereinafter.

The present invention provides a rhodanine derivative having the following general formula (I):

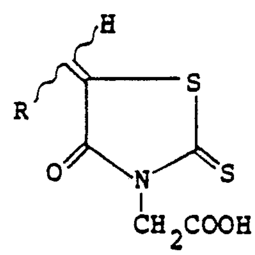

(I)

wherein R is an acyclic alkyl group having 2 to 10 carbon atoms which may be substituted by a halogen atom; an acyclic alkenyl group or polyenyl group having 1 to 4 olefinic double bonds having 2 to 19 carbon atoms which may be substituted by a halogen atom; a mono- or di-alkylamino group having 1 to 6 carbon atoms which may be substituted by a hydroxyl group; an alkenylamino group having 3 to 6 carbon atoms; a phenylamino group, the benzene ring of which may be substituted by a lower alkyl group, a lower alkoxyl group, phenyl group or a halogen atom; or a cyclic amino group which may contain in the ring an oxygen atom or a nitrogen atom to which a lower alkyl group is attached; and a nontoxic salt thereof.

The rhodanine derivative (I) of the present invention are classified into the following two groups:

The one is that represented by the following general formula (Ia):

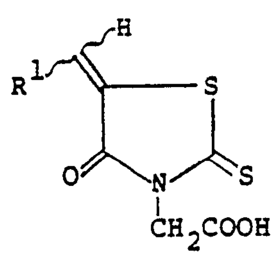

(Ia)

wherein $R^1$ is an acylic alkyl group having 2 to 10 carbon atoms which may be substituted by a halogen atom or an acyclic alkenyl group or polyenyl group having 1 to 4 olefinic double bonds having 2 to 19 carbon atoms which may be substituted by a halogen atom.

The other is that represented by the following general formula (Ib):

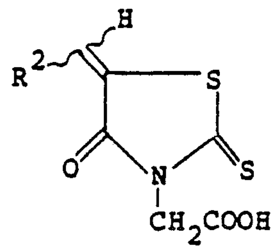

(Ib)

wherein $R^2$ is a mono- or di-alkylamino group having 1 to 6 carbon atoms which may be substituted by a hydroxyl group; an alkenylamino group having 3 to 6 carbon atoms; a phenylamino group, the benzene ring of which may be substituted by a lower alkyl group, a lower alkoxyl group, phenyl group or a halogen atom; or cyclic amino group which may contain in the ring an oxygen atom or a nitrogen atom to which a lower alkyl group is attached.

Examples of the halogen atom which may be contained in the acyclic alkyl or alkenyl group as the group $R^1$ in the formula (Ia) are fluorine, chlorine, bromine and iodine.

Examples of the acyclic alkyl group as the group $R^1$ in the formula (Ia) are, for instance, ethyl, 3-chloropropyl, t-butyl, n-decyl and n-pentyl groups. Examples of the acyclic alkenyl group or polyenyl group having 1 to 4 olefinic double bonds as the group $R^1$ in the formula (Ia) are, for instance, vinyl, 1-propenyl, 3-chloro-1-propenyl, 2-methyl-1-propenyl, 1-pentenyl, 1,3-pentadienyl, 2,6-dimethyl-1,5-heptadienyl and 2,6,10,14-tetramethyl-1,5,9,13-pentadecatetraenyl groups.

Examples of the alkylamino group as the group $R^2$ in the formula (Ib) are, for instance, methylamino, dimethylamino, 2-hydroxyethylamino, isobutylamino and hexylamino groups. Examples of the alkenylamino group as the group $R^2$ in the formula (Ib) for instance, allylamino, butenylamino, pentenylamino and hexenylamino groups. The phenylamino group as the group $R^2$ in the formula (Ib) may have on the benzene ring a lower alkyl group preferably having 1 to 4 carbon atoms, a lower alkoxy group preferably having 1 to 4 carbon atoms, phenyl group or a halogen atom such as fluorine, chlorine, bromine or iodine. Examples of the phenylamino group are, for instance, 2-biphenylamino, 3-methoxyphenylamino, 2-methoxyphenylamino, 3-chlorophenylamino, 4-bromophenylamino and m-tolylamino groups. The cyclic amino group as the group $R^2$ in the formula (Ib) may contain in the ring an oxygen atom or a nitrogen atom to which a lower alkyl group preferably having 1 to 3 carbon atoms is attahced, and preferably has a ring of 5 to 7 members. Examples of the cyclic amino group are morpholino, piperidino, 4-methylpiperazin-1-yl and 4-methylhomopiperazin-1-yl groups.

Examples of the nontoxic salts of the rhodanine derivative (I) are pharmaceutically acceptable salts, for instance, metal salts such as sodium salt, potassium salt, magnesium salt and calcium salt; salts with amine compounds represented by the general formula (V) mentioned below, such as methylamine, dimethylamine, ethanolamine, allylamine, isobutylamine, hexylamine, morpholine and methylpiperazine; mineral acid salts such as hydrochloride and sulfate.

The rhodanine derivative (I) includes any of trans form, cis form and mixture of these isomers.

The compounds represented by the general formula (I) and nontoxic salts thereof as provided by the present invention have potant platelet aggregation inhibiting activity which is equal to or better than that of Persantine (dipyridamole by generic name) which is an anti-platelet aggregation agent in widest use. At the same time, they have potent aldose reductase inhibiting activity and very low toxicity. Therefore, they can serve as excellent therapeutic agents for diabetic complications.

The preferred compounds among the compounds represented by the general formula (I) are shown in Tables 1 and 2. The compounds in Table 1 belong to the compound represented by the formula (Ia) and the compounds in Table 2 belong to the compound represented by the formula (Ib).

Table 1

| Compound No. | Formula | Name |
| --- | --- | --- |
| 1 | 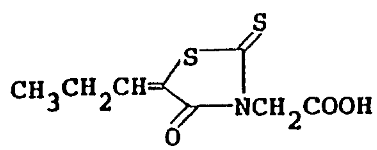 | 3-Carboxymethyl-5-propylidenerhodanine |
| 2 | 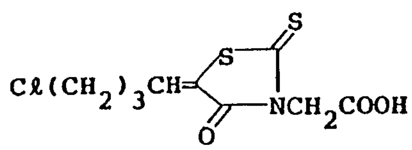 | 3-Carboxymethyl-5-(4-chlorobutylidene)rhodanine |
| 3 | 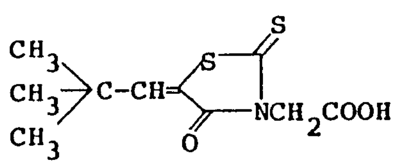 | 3-Carboxymethyl-5-(2,2-dimethylpropylidene)rhodanine |
| 4 | 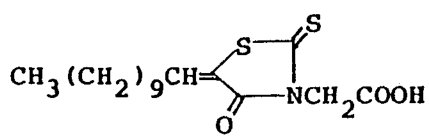 | 3-Carboxymethyl-5-undecylidenerhodanine |

- continued -

- continued-

| Compound No. | Formula | Name |
|---|---|---|
| 5 | 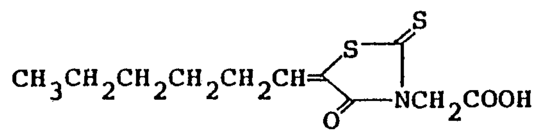 | 3-Carboxymethyl-5-n-hexylidenerhodanine |
| 6 | 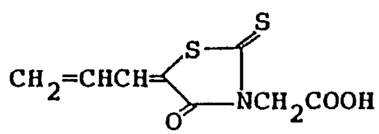 | 3-Carboxymethyl-5-allylidenerhodanine |
| 7 | 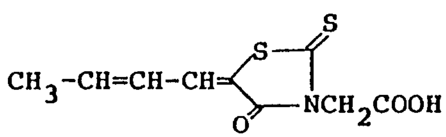 | 3-Carboxymethyl-5-(2-butenylidene)rhodanine |
| 8 | 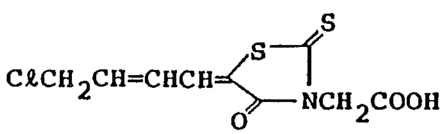 | 3-Carboxymethyl-5-(4-chloro-2-butenylidene)-rhodanine |

- continued -

- continued -

| Compound No. | Formula | Name |
|---|---|---|
| 9 | 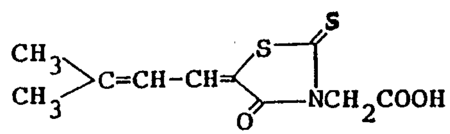 | 3-Carboxymethyl-5-(3-methyl-2-butenylidene)-rhodanine |
| 10 | 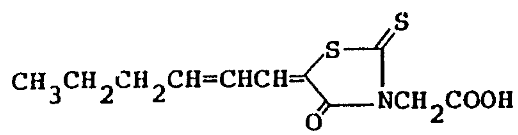 | 3-Carboxymethyl-5-(2-hexenylidene)rhodanine |
| 11 | 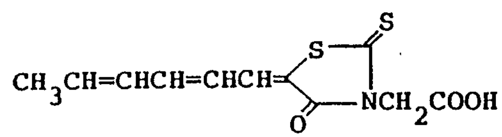 | 3-Carboxymethyl-5-(2,4-hexadienyliene)rhodanine |

- continued -

- continued -

| Compound No. | Formula | Name |
|---|---|---|
| 12 | 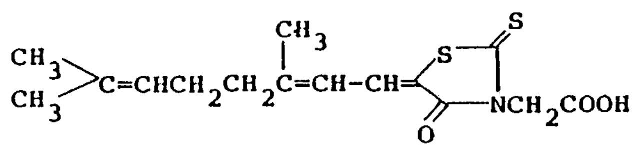 | 3-Carboyxymethyl-5-(3,7-dimethyl-2,6-octadienylidene)rhodanine |
| 13 | 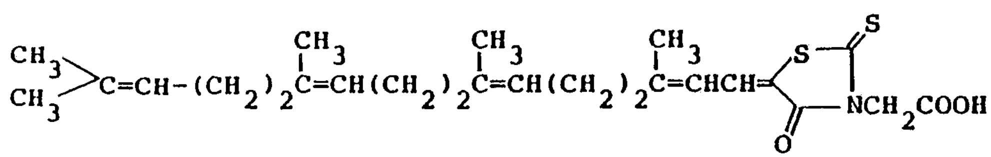 | 3-Carboxymethyl-5-(3,7,11,15-tetramethyl-2,6,10,14-hexadecatetraenylidene)-rhodanine |

Table 2

| Compound No. | Formula | Name |
| --- | --- | --- |
| 14 | 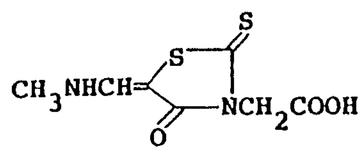 | 3-Carboxymethyl-5-methyl-aminomethylidenerhodanine |
| 15 | 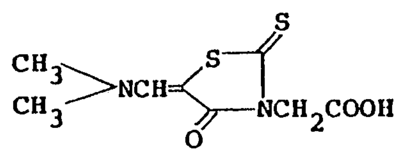 | 3-Carboxymethyl-5-dimethyl-aminomethylidenerhodanine |
| 16 | 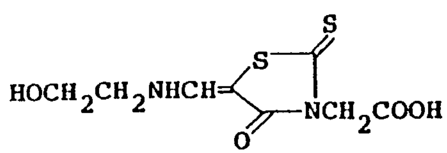 | 3-Carboxymethyl-5-hydroxy-ethylaminomethylidene-rhodanine |
| 17 | 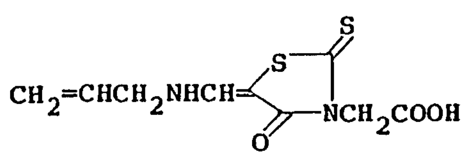 | 3-Carboxymethyl-5-allylamino-methylidenerhodanine |

- continued -

- continued -

| Compound No. | Formula | Name |
|---|---|---|
| 18 | 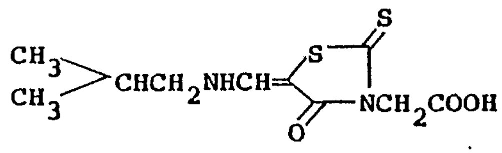 | 3-Carboxymethyl-5-isobutyl-aminomethylidenerhodanine |
| 19 | 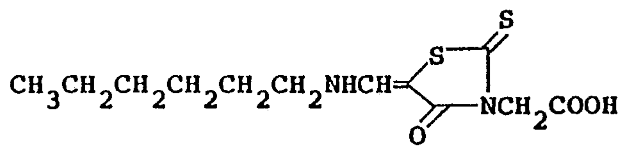 | 3-Carboxymethyl-5-hexylamino-methylidenerhodanine |
| 20 | 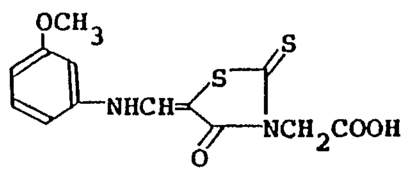 | 3-Carboxymethyl-5-m-methoxy-phenylaminomethylidene-rhodanine |
| 21 | 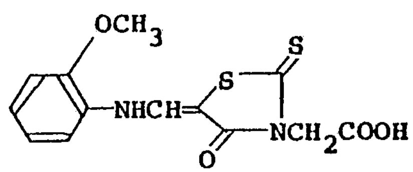 | 3-Carboxymethyl-5-o-methoxy-phenylaminomethylidene-rhodanine |

- continued -

- continued -

| Compound No. | Formula | Name |
|---|---|---|
| 22 | 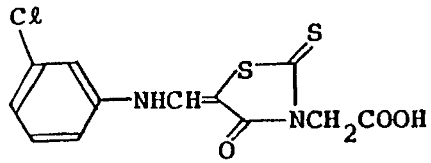 | 3-Carboxymethyl-5-m-chloro-phenylaminomethylidene-rhodanine |
| 23 | 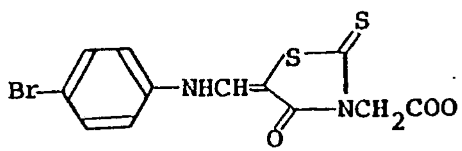 | 3-Carboxymethyl-5-p-bromo-phenylaminomethylidene-rhodanine |
| 24 | 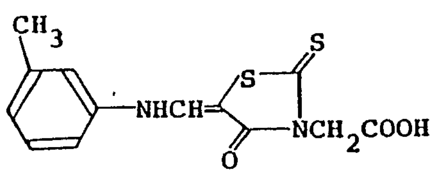 | 3-Carboxymethyl-5-m-methyl-phenylaminomethylidene-rhodanine |
| 25 | 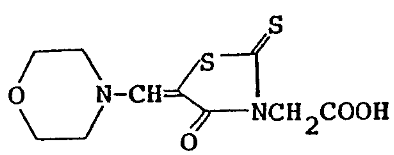 | 3-Carboxymethyl-5-morpholino-methylidenerhodanine |

- continued -

- continued -

| Compound No. | Formula | Name |
|---|---|---|
| 26 | 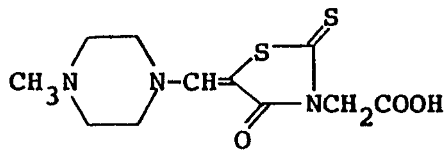 | 3-Carboxymethyl-5-N-methyl-piperazinomethylidene-rhodanine |
| 27 | 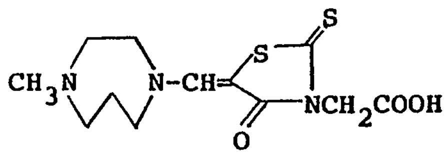 | 3-Carboxymethyl-5-N-methyl-homopiperazinomethylidene-rhodanine |
| 28 | 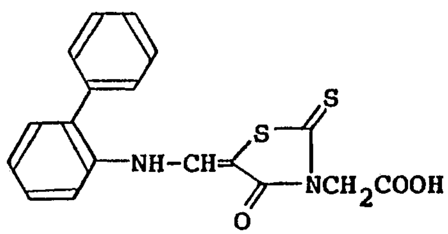 | 3-Carboxymethyl-5-(2-biphenylyl)aminomethylidene-rhodanine |

Japanese Patent Application Kokai Tokkyo Koho Nos. 57—28074 and 57—40478 disclose some rhodanine derivatives for use as aldose reductase inhibitors. However, between the rhodanine derivatives described in the above-cited publications and the rhodanine derivatives of the invention, there is a substantial difference in the substituent at position 5. Moreover, the rhodanine derivatives of the invention have platelet aggregation inhibiting activity. The publications cited above mention nothing of such activity.

Among the rhodanine derivatives of the invention, the 2-allylidene derivative (Compound No. 6), the 4-chloro-2-butenylidene derivative (Compound No. 8), the 2-butenylidene derivative (Compound No. 7), the 2-hexenylidene derivative (Compound No. 10), the 2,4-hexadienylidene derivative (Compound No. 11) and the 3,7,11,15-tetramethyl-2,6,10,14-hexadecatetraenylidene derivative (Compound No. 13) have a noticeably strong platelet aggregation inhibiting activity.

In addition, the rhodanine derivatives (Ib) and nontoxic salts thereof according to the present invention further contain a secondary or tertiary amine moiety in their molecule. The introduction of such amine moiety can increase the hydrophilic character and decrease the relative liposolubility, whereby the aldose reductase inhibiting activity *in vivo* can be prevented from decreasing as a result of protein binding and so forth [cf. Bird, A. E. and Marshall, A. C., Biochem. Pharmacol., *16*, 2275 (1967); Scholtan, W., Arznemittel-forschung, *18*, 505 (1968); Kitagawa, H., Noguchi, T. and Ito, R., Kusuri no Taisha (Drug Metabolism), page 87 (1971); Jusko, W. J. and Gretch, M., Drug Metabolism Reviews, *5*, 43 (1976)].

The rhodanine derivatives (I) of the present invention are prepared by the following processes.

The rhodanine derivatives represented by the general formula (Ia) are prepared by reacting 3-carboxymethylrhodanine having the following formula (II):

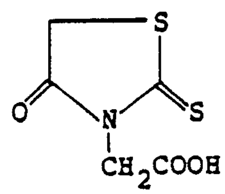

(II)

or a salt thereof with an aldehyde compound having the following general formula (III):

$$R^1—CHO \qquad (III)$$

wherein $R^1$ is an acyclic alkyl group having 1 to 9 carbon atoms which may be substituted by a halogen atom or an acyclic alkenyl group having 2 to 18 carbon atoms which may be substituted by a halogen atom.

Concretely, 3-carboxymethylrhodanine or its salt is dissolved into a solvent such as acetone, dimethylformamide (hereinafter referred to as "DMF), dimethyl sulfoxide (hereinafter referred to as "DMSO") or acetic acid. To the solution is added an alkali catalyst such as potassium carbonate, sodium hydroxide or sodium acetate. The amount of the catalyst used is preferably from 1 to 2 moles per 1 mole of 3-carboxymethylrhodanine. An aldehyde compound (III) is added to the mixture dropwise at room temperature or under heating at 50° to 90°C. The amount of the aldehyde compound used is preferably from 1 to 1.5 moles per 1 mole of 3-carboxymethylrhodanine. The obtained mixture is agitated at a temperature of from room temperature to 120°C for 1 to 24 hours. After the reaction is completed, the reaction mixture is poured into water and the resultant is extracted with an appropriate organic solvent such as ethyl acetate. The extract is purified in a usual manner such as silica gel column chromatography or recrystallization to give a rhodanine derivative (Ia).

The rhodanine derivatives represented by the general formula (Ib) are prepared by reacting a 3-carboxymethylrhodanine derivative having the following general formula (IV):

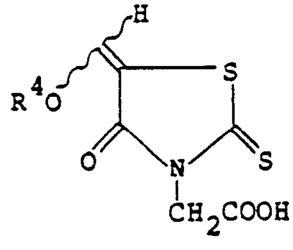

(IV)

wherein $R^4$ is a lower alkyl group, or a salt thereof with an amine compound having the following general formula (V):

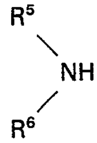

(V)

wherein $R^5$ is hydrogen atom or a lower alkyl group, and $R^6$ is an alkyl group having 1 to 6 carbon atoms which may be substituted by a hydroxyl group; an alkenyl group having 3 to 6 carbon atoms; phenyl group, the benzene ring of which may be substituted by a lower alkyl group, a lower alkoxyl group, phenyl group or a halogen atom; or $R^5$ and $R^6$ may be combined together with the nitrogen atom to which $R^5$ and $R^6$ attach to form a ring which may contain in the ring an oxygen atom or a nitrogen atom to which a lower alkyl group is attached.

In the above formula (IV), the lower alkyl group represented by $R^4$ is preferably alkyl groups having 1 to 4 carbon atoms, such as methyl, ethyl, propyl and butyl groups.

In the above formula (V), the lower alkyl group represented by $R^5$ is preferably alkyl groups having 1 to 4 carbon atoms, such as methyl, ethyl, n-propyl, isopropyl, n-butyl and isobutyl groups. The group represented by $R^6$ is alkyl groups having 1 to 6 carbon atoms which may be substituted by a hydroxyl group, such as methyl, ethyl, n-propyl, isopropyl, n-butyl, isobutyl, n-pentyl, n-hexyl and 2-hydroxyethyl; alkenyl groups having 3 to 6 carbon atoms, such as allyl, butenyl, pentenyl and hexenyl groups; and phenyl groups which may be substituted by a lower alkyl group preferably having 1 to 4 carbon atoms such as methyl, ethyl, propyl or butyl group, a lower alkoxyl group preferably having 1 to 4 carbon atoms such as methoxy, ethoxy, propoxy or butoxy group, phenyl group or a halogen atom such as fluorine, chlorine, bromine or iodine, for instance, 2-biphenylyl, 3-methoxyphenyl, 2-methoxyphenyl, 3-chlorophenyl, 4-bromophenyl and m-tolyl groups. Examples of the cyclic amine compound which $R^5$ and $R^6$ form together with the nitrogen atom to which $R^5$ and $R^6$ attach and may contain in the ring an oxygen atom or a nitrogen atom to which a lower alkyl group preferably having 1 to 3 carbon atoms, such as methyl, ethyl or propyl group, is attached are morpholine, piperidine, N-methylpiperazine and N-methylhomopiperazine.

The reaction of the compound (IV) with the amine compound (V) is conducted preferably in the following manner: The compound (IV) is dissolved in a polar protic solvent including a lower alcohol such as methanol, ethanol or propanol, or a polar aprotic solvent such as acetone, DMF or DMSO. In dissolving the compound (IV) in a solvent, the mixture may be heated somewhat. An amine compound (V) is added to the solution of the compound (IV). The amount of the amine compound (V) used is preferably from 1 to 2.5 mols per 1 mole of the compound (IV). The obtained mixture is agitated at a temperature of from room temperature to 90°C, preferably from 50° to 60°C, for 1 to 24 hours. After the reaction is completed, the reaction mixture is cooled to form a precipitate. The precipitate is separated by filtration and recrystallized from a solvent to give the rhodanine derivative (Ib). Examples of the solvent used are mixture of methanol and ether; mixture of ethyl acetate and petroleum ether; ethyl acetate; ethanol; mixture of acetone and petroleum ether; acetone; water; mixture of DMSO and water; and mixture of methanol, ethyl acetate and petroleum ether.

The compound (IV) used as a starting material is prepared, for instance, by dissolving 3-carboxymethyl-rhodanine represented by the formula (II) mentioned above or its salt in a solvent such as acetic anhydride, adding to the solution ethyl orthformate in an amount of 1 to 2 moles per 1 mole of the compound (II), agitating the resultant under reflux for 1 to 5 hours to effect the reaction, concentrating the reaction mixture, and purifying the resulting residue in a usual manner such as recrystallization or silica gel column chromatography.

The rhodanine derivatives and nontoxic salts thereof according to the invention are useful in the prevention or treatment of diabetic complications such as diseases of circulatory organs due to their excellent platelet aggregation inhibiting activity, and also useful in the prevention or treatment of nervous disturbance resulting from abnormal accumulation of polyols, diabetic retinopathy and cataract due to their excellent aldose reductase inhibiting activity. The effect on such prevention and treatment can be sufficiently exhibited by the dosage of about 0.05 to about 200 mg/day to adult.

The rhodanine derivatives (I) and nontoxic salt thereof can be formulated into pharmaceutical compositions in the form of tablets, capsules, injections, powders, pills, granules, suppositories and eye drops. Such preparations can be prepared in a usual manner using conventional pharmaceutically acceptable carriers. Examples of the carrier are, for instance, excipients, binders, diluents and lubricants. Typical examples thereof are lactose, starch, sugar, microcrystalline cellulose, magnesium stearate, silicon dioxide, talc, physiological salt solution and sterilized water.

The present invention is more specifically described and explained by means of the following Examples. It is to be understood that the present invention is not limited to the Examples, and various change and modifications may be made in the invention without departing from the spirit and scope thereof.

The abbreviations in the Examples mean the following:

MP: Melting point.

EA: Elementary analysis.

IR: Infrared absorption spectrum ($\nu_{max}^{KBr}$ $cm^{-1}$).

MS: Mass spectrum (20 eV, Direct).

NMR: Proton nuclear magnetic resonance spectrum (in DMSO-$d_6$, δ (ppm)).

In addition, the proton in the group represented by the formula "—CH=(Rh)" in NMR spectra means the proton of the group "—CH=" which is directly attached to the 5-position of 3-carboxymethylrhodanine.

Example 1

[3-Carboxymethyl-5-(2,2-dimethylpropylidene)rhodanine (Compound No. 3)].

Into 20 ml of dry DMF was dissolved 1.5 g (0.008 mole) of 3-carboxymethylrhodanine. To the solution was added 2.2 g (0.016 mole) of $K_2CO_3$ at a room temperature, and further 1 g (0.012 mole) of 2,2-dimethylpropanal was added dropwise at 60°C. After agitating at 60°C for 30 minutes, the reaction mixture was poured into water. The resultant was adjusted to pH 4 with a dilute hydrochloric acid and then extracted with ethyl acetate. The extract was purified through a silica gel column and recrystallized from a mixed solvent of ethyl acetate and ethanol to give 1.3 g of 3-carboxymethyl-5-(2,2-dimethylpropylidene)-rhodanine in the form of light yellow needle (yield: 63%).

The characteristic properties of the product are as follows:

MP: 160° to 163°C (from ethyl acetate-ethanol).

IR ($cm^{-1}$): 3300 to 3100 (COOH), 1730 (ring C=O).

MS (m/e): 259 ($M^+$), 241 ($M^+$—$H_2O$), 213 ($M^+$—COOH).

NMR (ppm): 1.17 (9H, s, $C(CH_3)_3$, 4.69 (2H, s, >$NCH_2$—), 6.88 (1H, s, —CH=), 9.81 (1H, bs, COOH).

EA for $C_{10}H_{13}O_3NS_2$ (MW=259.348):

Calcd. (%): C 46.31 H 5.05 N 5.40

Found (%): C 46.50 H 5.21 N 5.53.

Example 2

[3-Carboxymethyl-5-(2,4-hexadienylidene)rhodanine (Compound No. 11)].

4 Grams (0.021 mole) of 3-carboxymethylrhodanine was dissolved into 30 ml of acetic acid. To the solution were added 2 g (0.021 mole) of hexa-2,4-dienal and 3.8 g (0.046 mole) of sodium acetate, and the resultant was stirred under mild reflux for 5 hours. The obtained black reaction mixture was concentrated to about one-half its initial volume under reduced pressure. The residual mixture was poured into water to form precipitate. The precipitate was extracted with ethyl acetate and the extract was recrystallized from a mixed solvent of methylene chloride and ethyl acetate to give 4.1 g of 3-carboxymethyl-5-(2,4-hexadienylidene)rhodanine in the form of yellow prism (yield: 73%).

The characteristic properties of the product are as follows:

MP: 214° to 216°C (decomposition) (from methylene chloride-ethyl acetate).

IR ($cm^{-1}$): 3050 to 3200 (COOH), 1730 (ring C=O).

MS (m/e): 269 ($M^+$), 251 ($M^+$—OH), 223 ($M^+$—COOH).

NMR (ppm): 1.71 (3H, d, $CH_3$), 4.40 (2H, s, >$NCH_2$—), 5.60 to 6.70 (4H, m, —CH=CH—CH=CH—), 7.01 (1H, d, —CH=(Rh)), 8.30 (1H, bs, —COOH).

EA for $C_{11}H_{11}O_3NS_2$ (MW=269.344):

Calcd. (%): C 49.05 H 4.12 N 5.20

Found (%): C 48.90 H 4.19 N 5.35.

Example 3

[3-Carboxymethyl-5-propylidenerhodanine (Compound No. 1)].

The same procedures as in Example 2 except that propionaldehyde was used instead of hexa-2,4-dienal were repeated to give 3-carboxymethyl-5-propylidenerhodanine (yield: 67%).

The characteristic properties of the product are as follows:

MP: 138° to 140°C.

IR ($cm^{-1}$): 3150 (COOH), 1720 (ring C=O).

MS (m/e): 231 ($M^+$), 213 ($M^+$—OH), 185 ($M^+$—COOH).

NMR (ppm): 6.05 (3H, t, $CH_3$), 2.15 (2H, m, $CH_3C\mathit{H_2}$—), 4.38 (2H, s, >$NCH_2$—), 6.59 (1H, t, —CH=), 8.05 (1H, bs, COOH).

EA for $C_8H_9NO_3S_2$ (MW=231.294):

Calcd. (%): C 41.54 H 3.92 N 6.06

Found (%): C 41.68 H 3.99 N 6.28.

Example 4

[3-Carboxymethyl-5-(4-chlorobutylidene)rhodanine (Compound No. 2)].

The same procedures as in Example 2 except that 4-chlorobutyraldehyde was used instead of hexa-2,4-dienal were repeated to give the desired product (yield: 76%).

The characteristic properties of the product are as follows:

MP: 155° to 157°C.

IR ($cm^{-1}$): 3180 (COOH), 1734 (ring C=O), 1635 (COOH).

MS (m/e): 279 ($M^+$), 261 ($M^+$—OH), 243 ($M^+$—HCl), 233 ($M^+$—COOH).

NMR (ppm): 1.90 (2H, m, $ClCH_2C\mathit{H_2}CH_2$—), 2.32 (2H, q, —$\mathit{CH_2}$—CH=), 3.54 (2H, t, $ClCH_2$—), 4.51 (2H, s, >$NCH_2$—), 6.78 (1H, d, $CH_2C\mathit{H}$=), 8.10 (1H, bs, COOH).

EA for $C_9H_{10}NO_3S_2Cl$ (MW=279.766):

Calcd. (%): C 38.64 H 3.60 N 5.01

Found (%): C 38.51 H 3.77 N 5.21.

Example 5

[3-Carboxymethyl-5-undecylidenerhodanine (Compound No. 4)].

The same procedures as in Example 2 except that undecanealdehyde was used instead of hexa-2,4-dienal where repeated to give the desired compound (yield: 79%).

The characteristic properties of the product are as follows:

MP: 61° to 63°C.

IR ($cm^{-1}$): 2950 (COOH), 1730 (ring C=O).

MS (m/e): 343 ($M^+$), 204 ($M^+$—$CH_3(CH_2)_8$).

NMR (ppm): 0.82 (3H, bd, $CH_3$), 1.20 (16H, bs, $CH_3CH_2(CH_2)_8$—), 2.16 (2H, bq, $CH_3CH_2$—), 4.49 (2H, s, >$NCH_2$—), 6.72 (1H, t, —CH=), 8.20 (1H, bs, COOH)

EA for $C_{16}H_{25}NO_3S_2$ (MW=343.510):

| | | | |
|---|---|---|---|
| Calcd. (%): | C 55.95 | H 7.34 | N 4.08 |
| Found (%): | C 56.16 | H 7.19 | N 4.20. |

Example 6

[3-Carboxymethyl-5-n-hexylidenerhodanine (Compound No. 5)].

The same procedures as in Example 2 except that hexanal was used instead of hexa-2,4-dienal were repeated to give the desired compound (yield: 75%).

The characteristic properties of the product are as follows:

MP: 136° to 140°C (from n-hexane).

IR ($cm^{-1}$): 3430 (COOH), 1715 (ring C=O).

MS (m/e): 273 ($M^+$), 227 ($M^+$—COOH), 204 ($M^+$—$CH_3(CH_2)_4$).

NMR (ppm): 0.84 (3H, bt, $CH_3$), 1.05 to 1.70 (6H, m, $CH_3(CH_2)_3$—), 2.18 (2H, bq, —$CH_2$—CH=), 4.56 (2H, s, >$NCH_2$—), 6.98 (1H, t, —CH=), 8.70 (1H, bs, COOH).

EA for $C_{11}H_{15}NO_3S_2$ (MW=273.360):

| | | | |
|---|---|---|---|
| Calcd. (%): | C 48.33 | H 5.53 | N 5.12 |
| Found (%): | C 48.22 | H 5.60 | N 5.01. |

Example 7

[3-Carboxymethyl-5-allylidenerhodanine (Compound No. 6)].

The same procedures as in Example 2 except that acrolein was used instead of hexa-2,4-dienal were repeated to give the desired compound (yield: 67%).

The characteristic properties of the product are as follows:

MP: 144° to 146°C.

IR ($cm^{-1}$): 3430 (COOH), 1735 (ring C=O).

MS (m/e): 229 ($M^+$), 112 ($M^+$—C(=S)—$NCH_2COOH$).

NMR (ppm): 4.39 (2H, s, >$NCH_2$—), 5.50 (1H, dd, 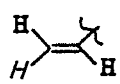 ),

5.69 (1H, dd, 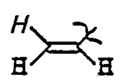 ), 6.10 (1H, m, $H_2C=CH$—), 6.98 (1H, d, —CH=(Rh)),

8.52 (1H, bs, COOH)

EA for $C_8H_7NO_3S_2$ (MW=229.278):

| | | | |
|---|---|---|---|
| Calcd. (%): | C 41.91 | H 3.08 | N 5.11 |
| Found (%): | C 41.76 | H 2.88 | N 6.25. |

Example 8

[3-Carboxymethyl-5-(2-butenylidene)rhodanine (Compound No. 7)].

The same procedures as in Example 2 except that 2-butenal was used instead of hexa-2,4-dienal were repeated to give the desired compound (yield: 80%).

The characteristic properties of the product are as follows:

MP: 216° to 218°C (from ethanol-water).

IR ($cm^{-1}$): 3140 (COOH), 1737 (ring C=O), 1686 (COOH).

MS (m/e): 243 ($M^+$), 225 ($M^+$—OH), 197 ($M^+$—COOH).

NMR (ppm): 1.82 (3H, d, $CH_3$), 4.45 (2H, s, >$NCH_2$—), 6.05 (1H, dd, —CH=CH—), 6.35 (1H, m, $CH_3CH$=), 7.18 (1H, d, —CH=(Rh)), 8.03 (1H, bs, COOH)

EA for $C_9H_9NO_3S_2$ (MW=243.290):

| | | | |
|---|---|---|---|
| Calcd. (%): | C 44.43 | H 3.73 | N 5.76 |
| Found (%): | C 44.35 | H 3.83 | N 5.64. |

Example 9

[3-Carboxymethyl-5-(4-chloro-2-butenylidene)rhodanine (Compound No. 8)].

The same procedures as in Example 2 except that 4-chloro-2-butenal was used instead of hexa-2,4-dienal were repeated to give the desired compound (yield: 85%).

The characteristic properties of the product are as follows:

MP: 205° to 207°C (purified by column chromatography).

IR ($cm^{-1}$): 3150 (COOH), 1739 (ring C=O), 1677 (COOH).

MS (m/e): 277 ($M^+$), 259 ($M^+$—OH), 243 ($M^+$—Cl), 231 ($M^+$—COOH), 228 ($M^+$—$ClCH_2$).

NMR (ppm): 4.20 (2H, d, $ClCH_2$—), 4.48 (2H, s, >$NCH_2$—), 6.30 to 6.54 (2H, m, —CH=CH—), 7.26 (1H, d, —$CH_2$=(Rh)).

EA for $C_9H_8NO_3S_2Cl$ (MW=277.750).

Calcd. (%): C 38.92 H 2.90 N 5.04

Found (%): C 38.69 H 2.83 N 5.14.

Example 10

[3-Carboxymethyl-5-(3-methyl-2-butenylidene)rhodanine (Compound No. 9)].

The same procedure as in Example 2 except that 3-methyl-2-butenal was used instead of hexa-2,4-dienal were repeated to give the desired compound (yield: 57%).

The characteristic properties of the product are as follows:

MP: 206° to 210°C (decomposition).

IR ($cm^{-1}$): 3220 (COOH), 1737 (ring C=O), 1697 (COOH).

MS (m/e): 257 ($M^+$), 239 ($M^+$—OH), 212 ($M^+$—COOH).

NMR (ppm): 1.88 (6H, s, ($CH_3$—) X 2), 4.48 (2H, s, >$NCH_2$—), 5.82 (1H, d, $(CH_3)_2C=CH$—), 7.30 (1H, d, —CH=(Rh)), 8.80 (1H, bd, COOH).

EA for $C_{10}H_{11}NO_3S_2$ (MW=257.320):

Calcd. (%): C 46.67 H 4.31 N 5.44

Found (%): C 46.70 H 4.31 N 5.40.

Example 11

[3-Carboxymethyl-5-(2-hexenylidene)rhodanine (Compound No. 10)].

The same procedures as in Example 2 except that 2-hexenal was used instead of hexa-2,4-dienal were repeated to give the desired compound (yield: 71%).

The characteristic properties of the product are as follows:

MP: 188° to 190°C.

IR ($cm^{-1}$): 3100 (COOH), 1724 (ring C=O).

MS (m/e): 271 ($M^+$), 253 ($M^+$—OH), 225 ($M^+$—COOH).

NMR (ppm): 0.85 (3H, t, $CH_3$), 1.40 (2H, m, $CH_3CH_2$—), 2.14 (2H, q, $CH_3CH_2CH_2$—), 4.49 (2H, s, >$NCH_2$—), 5.98 (1H, m, olefinic proton), 6.36 (1H, m, olefinic proton), 7.10 (1H, d, —CH=(Rh)), 9.10 (1H, bs, COOH).

EA for $C_{11}H_{13}NO_3S_2$ (MW=271.334):

Calcd. (%): C 48.69 H 4.83 N 5.16

Found (%): C 48.44 H 5.03 N 5.19.

Example 12

[3-Carboxymethyl-5-(3,7-dimethyl-2,6-octadienylidene)-rhodanine (Compound No. 12)].

The same procedures as in Example 2 except that 3,7-dimethyl-2,6-octadienal was used instead of hexa-2,4-dienal were repeated to give the desired compound (yield: 65%).

The characteristic properties of the product are as follows:

MP: 95° to 98°C.

IR ($cm^{-1}$): 3400 (COOH).

MS (m/e): 325 ($M^+$), 282 ($M^+$—$(CH_3)_2$—CH), 257 ($M^+$—$(CH_3)_2CH=CHCH_2$).

NMR (ppm): 1.55 (3H, bs, terminal $CH_3$), 1.58 (3H, bs, terminal $CH_3$), 1.85 (4H, m, —$CH_2CH_2$—), 1.92 (3H, s, $-\underset{\displaystyle CH_3}{\underset{|}{C}}=$), 4.85 (2H, s, >$NCH_2$—), 4.98 (1H, m, $(CH_3)_2C=CH$—),

5.88 (0.5H, bd, $-\overset{\displaystyle CH_3}{\overset{|}{C}}=CH-CH=$), 6.01 (0.5H, bd, $-\overset{\displaystyle CH_3}{\overset{|}{C}}=CH-CH=$),

7.38 (0.5H, bd, —CH=(Rh)), 7.50 (0.5H, bd, —CH=(Rh)), 8.10 (1H, bs, COOH)

EA for $C_{15}H_{19}NO_3S_2$ (MW=325.430)

Calcd. (%): C 55.36 H 5.89 N 4.30

Found (%): C 55.50 H 5.95 N 4.20.

### Example 13

[3-Carboxymethyl-5-(3,7,11,15-tetramethyl-2,6,10,14-hexadecatetraenylidene)rhodanine (Compound No. 13)].

The same procedures as in Example 2 except that 3,7,11,15-tetramethyl-2,6,10,14-hexadecatetraenal was used instead of hexa-2,4-dienal were repeated to give the desired compound (yield: 54%).

The characteristic properties of the product are as follows:

MP: 35° to 37°C.

IR ($cm^{-1}$): 3450 (COOH), 1730 (ring C=O), 1720 (COOH).

MS (m/e): 461 ($M^+$), 416 ($M^+$—COOH), 257 ($M^+$-farnesyl group).

NMR (ppm): 1.48 (15H, bs, ($CH_3$—) X 5), 1.87 (12H, m, (—$CH_2CH_2$—) X 3), 4.48 (2H, bs, >$NCH_2$—),

4.84 (3H, m, ( 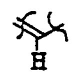 ) X 3), 5.76 (1H, bd, 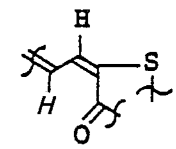 ),

7.30 (1H, d, —CH=(Rh)), 7.99 (1H, bs, COOH)

EA for $C_{25}H_{35}NO_3S_2$ (MW=461.660)

| | | | |
|---|---|---|---|
| Calcd. (%): | C 65.04 | H 7.64 | N 3.03 |
| Found (%): | C 65.31 | H 7.80 | N 2.95. |

### Example 14

[3-Carboxymethyl-5-(4-chloro-2-butenylidene)rhodanine sodium salt (sodium salt of Compound No. 8)].

3000 Milligrams of 3-carboxymethyl-5-(4-chloro-2-butenylidene)rhodanine obtained in Example 9 was dissolved into 10.8 ml of 0.1N sodium hydroxide aqueous solution and the resulting solution was freeze-dried to yield quantitatively the desired compound in the form of yellow powder.

The characteristic properties of the product are as follows:

MP: 270° to 273°C (decomposition).

IR ($cm^{-1}$): 3130, 1735, 1682.

The sodium salts of Compound Nos. 1 to 7 and 9 to 12 were obtained in the same manner as described above.

### Example 15

[3-Carboxymethyl-5-(4-chloro-butenylidene)rhodanine potassium salt (potassium salt of Compound No. 8)].

300 Milligrams of 3-carboxymethyl-5-(4-chloro-2-butenylidene)rhodanine was dissolved into 10.8 ml of 0.1N potassium hydroxide aqueous solution and the resulting solution was freeze-dried to yield quantitatively the desired compound in the form of light vermilion powder.

The characteristic properties of the product are as follows:

MP: 280° to 287°C (decomposition).

IR ($cm^{-1}$): 3130, 1736, 1679.

The potassium salts of Compound Nos. 1 to 7 and 9 to 13 were obtained in the same manner as described above.

### Reference Example

[3-Carboxymethyl-5-ethoxymethylidenerhodanine].

A mixture of 36 g (0.189 mole) of 3-carboxymethylrhodanine, 33.6 g (0.225 mole) of ethyl orthoformate and 225 ml of acetic anhydride was subjected to reaction under reflux for about 1 to 2 hours and then the reaction mixture was concentrated. A precipitate was formed by the addition of a small amount of chloroform to the residue. The precipitate was separated and recrystallized from ethyl acetate to give 29.2 g of 3-carboxymethyl-5-ethoxymethylidenerhodanine in the form of brownish white crystal (yield: 62.5%).

The characteristic properties of the product are as follows:

MP: 190° to 192°C.

IR ($cm^{-1}$): 3100 to 2900 (CH), 2600 to 2400 (COOH), 1730 (ring C=O), 1680 (COOH).

MS (m/e): 247 ($M^+$), 219 ($M^+$—$C_2H_4$)

NMR (ppm): 1.25 (3H, t, —$CH_3$), 4.15 (2H, q, —$CH_2$—), 4.40 (2H, s, —$CH_2COO$—), 7.60 (1H, s, —CH=).

### Example 16

[3-Carboxymethyl-5-methylaminomethylidenerhodanine (Compound No. 14)].

A mixture of 0.68 g (0.010 mole) of methylamine hydrochloride and 5 ml of ethanol, which was neutralized with 1.0 g (0.010 mole) of triethanolamine, was added to a mixture of 1.2 g (0.005 mole) of 3-carboxymethyl-5-ethoxymethylidenerhodanine and 10 ml of ethanol. The reaction was conducted at a temperature of 50° to 60°C for 1 hour. The reaction mixture was concentrated and the resulting residue was recrystallized from ethanol to give 1.0 g of 3-carboxymethyl-5-methylaminomethylidenerhodanine methylamine salt (yield: 79.2%).

The salt was dissolved into water and the resulting solution was made neutral or weekly acidic with a dilute hydrochloric acid. The formed precipitate was separated by filtration and recrystallized from a mixture of methanol, ethyl acetate and petroleum ether (5/3/1 by volume to give 0.56 g of 3-carboxymethyl-5-methylaminomethylidenerhodamine in the form of brown powdered crystal (yield: 46.7%).

The characteristic properties of the product are as follows:

MP: 247° to 249°C (decomposition).

IR ($cm^{-1}$): 3300 to 2850 (NH, CH), 2700 to 2450 (COOH), 1730 (ring C=O), 1690 (COOH).

MS (m/e): 232 ($M^+$), 188 ($M^+$—COO).

NMR (ppm): 2.85 (3H, d, $-\overset{|}{N}-CH_3$), 4.3 (2H, s, $-CH_2COO-$), 7.25 (1H, d, —CH=), 7.90 (1H, d, —NH—).

EA: shown in Table 3.

Example 17

[3-Carboxymethyl-5-dimethylaminomethylidenerhodanine (Compound No. 15).

The same procedures as in Example 16 except that dimethylamine hydrochloride was used instead of methylamine hydrochloride were repeated to give 3-carboxymethyl-5-dimethylaminomethylidenerhodanine in the form of reddish brown crystal (yield: 73.3%).

The characteristic properties of the product are as follows:

MP: 202° to 204°C (decomposition).

IR ($cm^{-1}$): 3250 to 2800 (CH), 2650 to 2400 (COOH), 1730 (ring C=O), 1680 (COOH).

MS (m/e): 246 ($M^+$).

NMR (ppm): 3.00 (6H, s, $-N\genfrac{}{}{0pt}{}{/ CH_3}{\backslash CH_3}$), 4.30 (2H, s, $-CH_2COO-$), 7.30 (1H, s, —CH=).

EA: shown in Table 3.

Example 18

[Ethanolamine salt of 3-Carboxymethyl-5-hydroxyethylaminomethylidenerhodanine (Compound No. 16)].

0.62 Gram of monoethanolamine was added to a mixture of 1.2 g (0.005 mole) of 3-carboxymethyl-5-ethoxymethylidenerhodanine and 10 ml of ethanol. The reaction was carried out at a temperature of 50° to 60°C for 1 hour. The formed precipitate was separated by filtration and recrystallized from a mixture of methanol and ether (7/3 by volume) to give 0.85 g of 3-carboxymethyl-5-hydroxyethylaminomethylidenerhodanine ethanolamine salt in the form of light brown crystal (yield: 53.1%).

The characteristic properties of the product are as follows:

MP: 170° to 172°C (decomposition).

IR ($cm^{-1}$): 3400 to 2800 (OH, NH, CH), 2600 to 2400 (COOH), 1700 (ring C=O).

NMR (ppm): 2.95 (2H, t, $-CH_2NH_2$), 3.2 to 3.7 (6H, m, $HOCH_2CH_2NH-$, $HOCH_2CH_2NH_2$), 4.25 (2H, s, $-CH_2COO-$), 7.4 (1H, s, —CH=).

EA: shown in Table 3.

Example 19

[3-Carboxymethyl-5-allylaminomethylidenerhodanine (Compound No. 17)].

0.3 Gram (0.005 mole) of allylamine was added to a mixture of 1.2 g (0.005 mole) of 3-carboxymethyl-5-ethoxymethylidenerhodanine and 10 ml of ethanol, and the resulting mixture was subjected to reaction at a temperature of 50° to 60°C for 1 hour. The reaction mixture was cooled and the formed precipitate was separated by filtration and recrystallized from a mixture of methanol and ether (7/3 by volume) to give 0.68 g of 3-carboxymethyl-5-allylaminomethylidenerhodanine in the form of yellow crystal (yield: 52.6%).

The characteristic properties of the product are as follows:

MP: 170° to 172°C (decomposition).

IR ($cm^{-1}$): 3300 to 2850 (NH, CH), 2600 to 2400 (COOH), 1730 (ring C=O), 1685 (COOH).

MS (m/e): 258 ($M^+$).

NMR (ppm): 3.75 (2H, d, $=CHCH_2\overset{|}{N}-$), 4.23 (2H, s, $-CH_2COO-$), 5.00 (2H, d, $CH_2=$), 5.43 to 5.70 (1H, m, $CH_2=CHCH_2-$), 7.30 (1H, s, —CH=).

EA: shown in Table 3.

Example 20

[3-Carboxymethyl-5-isobutylaminomethylidenerhodanine (Compound No. 18)].

0.74 Gram (0.01 mole) of isobutylamine was added to a mixture of 1.2 g (0.005 mole) of 3-carboxymethyl-5-ethoxymethylidenerhodanine and 10 ml of ethanol, and the resulting mixture was subjected to reaction at a temperature of 50° to 60°C for 1 hour. The reaction mixture was concentrated and the resulting residue was dried and dissolved into water. The solution was made neutral or weakly acidic

with a dilute hydrochloric acid and the formed precipitate was separated by filtration. The precipitate was washed with water, dried and recrystallized from a mixture of ethyl acetate and petroleum ether (4/1 by volume) to give 0.9 g of 3-carboxymethyl-5-isobutylaminomethylidenerhodanine in the form of yellowish red crystal (yield: 64.3 %).

The characteristic properties of the product are as follows:

MP: 174° to 176°C (decomposition).

IR ($cm^{-1}$): 3300 to 2850 (NH, CH), 2650 to 2450 (COOH), 1720 (ring C=O), 1680 (COOH).

MS (m/e): 274 ($M^+$), 230 ($M^+$—COO).

NMR (ppm): 0.9 (6H, d, 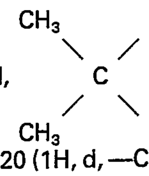 ), 1.40 to 1.85 (1H, m, >CH—), 2.95 (2H, t, —$CH_2$NH—), 4.23 (2H, s, —$CH_2$COO—), 7.20 (1H, d, —CH=), 8.00 (1H, d, —NH—).

EA: shown in Table 3.

Example 21

[3-Carboxymethyl-5-hexylaminomethylidenerhodanine (Compound No. 19)].

The same procedures as in Example 20 except that hexylamine was used instead of isobutylamine were repeated to give hexylamine salt of 3-carboxymethyl-5-hexylaminomethylidenerhodanine in the form of yellow crystal by recrystallization from ethyl acetate.

The characteristic properties of the product are as follows:

MP: 139° to 141°C (decomposition).

NMR (ppm): 0.90 (6H, t, (—$CH_3$) X 2) 1.00 to 1.60 (16H, m, (—$CH_2$—) X 8), 2.60 (2H, t, $NH_2CH_2CH_2$—), 3.15 (2H, t, —$NHCH_2CH_2$—), 4.10 (2H, s, —$CH_2$COO—), 7.18 (1H, s, —CH=).

The above salt was dissolved into water and the resulting solution was treated in the same manner as in Example 20. The obtained product was recrystallized from a mixture of ethyl acetate and petroleum ether (4/1 by volume) to give 0.9 g of 3-carboxymethyl-5-hexylaminomethylidenerhodanine in the form of yellow crystal (yield: 60%).

The characteristic properties of the product are as follows:

MP: 243° to 245°C (decomposition).

IR ($cm^{-1}$): 3300 to 2850 (NH, CH), 2600 to 2400 (COOH), 1710 (ring C=O), 1690 (COOH).

MS (m/e): 302 ($M^+$).

NMR (ppm): 0.85 (3H, t, $CH_3$—), 1.1 to 1.6 (8H, m, (—$CH_2$—) X 4), 3.0 to 3.3 (2H, m, —$NHCH_2CH_2$—), 4.23 (2H, s, —$CH_2$COO—), 7.3 (1H, s, —CH=), 7.8 to 8.2 (1H, broad, —NH—).

EA: shown in Table 3.

Example 22

[3-Carboxymethyl-5-m-methoxyphenylaminomethylidenerhodanine (Compound No. 20)].

The same procedures as in Example 19 except that m-anisidine was used instead of allylamine were repeated to give 1.1 g of 3-carboxymethyl-5-m-methoxyphenylaminomethylidenerhodanine by recrystallization from ethanol in the form of yellow crystal (yield: 68.8%).

The characteristic properties of the product are as follows:

MP: 240° to 242°C (decomposition).

IR ($cm^{-1}$): 3350 to 2800 (NH, CH), 2700 to 2500 (COOH), 1740 (ring C=O), 1680 (COOH).

MS (m/e): 324 ($M^+$).

NMR (ppm): 3.58 (3H, s, —$OCH_3$—), 4.40 (2H, s, —$CH_2$COO—), 6.20 to 6.97 (4H, m, aromatic proton), 7.65 (1H, d, —CH=), 9.8 (1H, d, —NH—)

EA: shown in Table 3.

Example 23

[3-Carboxymethyl-5-o-methoxyphenylaminomethylidenerhodanine (Compound No. 21)].

The same procedures as in Example 19 except that o-anisidine was used instead of allylamine and the reaction was conducted at a room temperature for 4 hours were repeated to give 1.0 g of 3-carboxymethyl-5-o-methoxyphenylaminomethylidenerhodanine in the form of yellow fine needle by recrystallization from ethanol (yield: 62.5%).

The characteristic properties of the product are as follows:

MP: 244° to 246°C (decomposition).

IR ($cm^{-1}$): 3300 to 2850 (NH, CH), 2700 to 2450 (COOH), 1720 (ring C=O), 1675 (COOH).

MS (m/e): 324 ($M^+$).

NMR (ppm): 3.60 (3H, s, —$OCH_3$), 4.36 (2H, s, —$CH_2$COO—), 6.50 to 7.10 (4H, m, aromatic proton), 7.55 (1H, d, —CH=), 9.58 (1H, d, —NH—).

EA: shown in Table 3.

Example 24

[3-Carboxymethyl-5-m-chlorophenylaminomethylidenerhodanine (Compound No. 22)].

The same procedures as in Example 19 except that m-chloroaniline was used instead of allylamine were repeated to give 1.2 g of 3-carboxymethyl-5-m-chlorophenylaminomethylidenerhodanine in the form

of yellow crystal by recrystallization from a mixture of acetone and petroleum ether (4/1 by volume) (yield: 73.2%).

The characteristic properties of the product are as follows:

MP: 253° to 255°C (decomposition).

IR ($cm^{-1}$): 3350 to 2850 (NH, CH), 2650 to 2350 (COOH), 1720 (ring C=O), 1670 (COOH).

MS (m/e): 328 ($M^+$), 284 ($M^+$—COO—).

NMR (ppm): 4.40 (2H, s, —$CH_2$COO), 6.68 to 7.15 (4H, m, aromatic, proton), 7.7 (1H, d, —CH=), 9.9 (1H, d, —NH—).

EA: shown in Table 3.

## Example 25

[3-Carboxymethyl-5-p-bromophenylaminomethylidenerhodanine (Compound No. 23)].

The same procedures as in Example 20 except that p-bromoaniline was used instead of isobutylamine were repeated to give 0.84 g of 3-carboxymethyl-5-p-bromophenylaminomethylidenerhodanine in the form of yellow crystal (yield: 44.0%).

The characteristic properties of the product are as follows:

MP: 264° to 266°C (decomposition).

IR ($cm^{-1}$): 3350 to 2850 (NH, CH), 2600 to 2350 (COOH), 1730 (ring C=O), 1670 (COOH).

MS (m/e): 373 ($M^+$), 375 ($M^+ + 2$).

NMR (ppm): 4.38 (2H, s, —$CH_2$COO—), 6.85 (2H, d, aromatic proton), 7.10 (2H, d, aromatic proton), 7.65 (1H, d, —CH=), 9.90 (1H, broad, —NH—).

EA: shown in Table 3.

## Example 26

[3-Carboxymethyl-5-m-methylphenylaminomethylidenerhodanine (Compound No. 24)].

The same procedures as in Example 20 except that m-toluidine was used instead of isobutylamine to give 1.15 g of 3-carboxymethyl-5-m-methylphenylaminomethylidenerhodanine in the form of yellow crystal by recrystallization from acetone (yield: 74.7%).

The characteristic properties of the product are as follows:

MP: 261° to 263°C (decomposition).

IR ($cm^{-1}$): 3350 to 2850 (NH, CH), 2650 to 2400 (COOH), 1740 (ring C=O), 1680 (COOH).

MS (m/e): 308 ($M^+$).

NMR (ppm): 2.30 (2H, s, —$CH_3$), 4.40 (2H, s, —$CH_2$COO—), 6.60 to 7.20 (4H, m, aroamtic proton), 7.70 (1H, d, —CH=), 9.90 (1H, broad, —NH—).

EA: shown in Table 3.

## Example 27

[3-Carboxymethyl-5-morpholinomethylidenerhodanine (Compound No. 25)].

The same procedures as in Example 20 except that morpholine was used instead of isobutylamine were repeated to give morpholine salt of 3-carboxymethyl-5-morpholinomethylidenerhodanine in the form of light yellow crystal by recrystallization from ethanol.

The characteristic properties of the product are as follows:

MP: 208° to 210°C (decomposition).

NMR (ppm): 2.85 (4H, t, 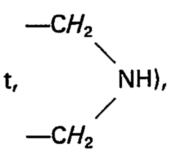), 3.30 to 3.60 (12H, m, (—$CH_2$—) X 6), 4.20 (2H, s, —$CH_2$COO—), 7.30 (1H, s, —CH=).

The above salt was dissolved into water and the resulting soltuion was treated in the same manner as in Example 20 to give 0.97 g of 3-carboxymethyl-5-morpholinomethylidenerhodanine in the form of light yellowish red crystal (yield: 67.2%).

The characteristic properties of the product are as follows:

MP: 293° to 295°C (decomposition).

IR ($cm^{-1}$): 3150 to 2850 (CH), 2650 to 2400 (COOH), 1720 (ring C=O), 1665 (COOH).

MS (m/e) 288 ($M^+$), 244 ($M^+$—COO).

NMR (ppm): 3.30 to 3.55 (8H, m, (—$CH_2$—) X 4), 4.30 (2H, s, —$CH_2$COO—), 7.30 (1H, s, —CH=).

EA: shown in Table 3.

## Example 28

[3-Carboxymethyl-5-N-methylpiperazinomethylidenerhodanine (Compound No. 26)].

The same procedures as in Example 19 except that N-methylpiperazine was used instead of allylamine were repeated to give 1.1 g of 3-carboxymethyl-5-N-methylpiperazinomethylidenerhodanine in the form of light yellowish red crystal by recrystallization from water (yield: 70.0%).

The characteristic properties of the product are as follows:
MP: 250° to 252°C (decomposition).
IR ($cm^{-1}$): 3350 to 2850 (CH), 2700 to 2300 (COOH), 1720 (ring C=O), 1670 (COOH).
MS (m/e): 301 ($M^+$).
NMR (ppm): 2.33 (3H, s, $CH_3$—N<), 2.40 to 2.8 (4H, m, 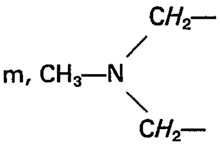 ),
3.40 to 3.8 (4H, m, 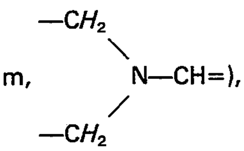 ), 4.30 (2H, s, —$CH_2$COO—), 7.40 (1H, s, —CH=).
EA: shown in Table 3.

Example 29

[3-Carboxymethyl-5-N-methylhomopiperazinomethylidenerhodanine (Compound No. 27)].

The same procedures as in Example 19 except that N-methylhomopiperazine was used instead of allylamine were repeated to give 1.3 g of 3-carboxymethyl-5-N-methylhomopoperazinomethylidenerhodanine in the form of light brown powder by recrystallization from a mixture of dimethylsulfoxide and water (9/1 by volume) (yield: 82.3%).

The characteristic properties of the product are as follows:
MP: 244° to 246°C (decomposition).
IR ($cm^{-1}$): 3350 to 2850 (CH), 2700 to 2300 (COOH), 1725 (ring C=O), 1670 (COOH).
MS (m/e): 315 ($M^+$).
NMR (ppm): 1.7 to 2.1 (2H, m, 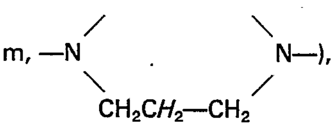 ), 2.33 (3H, s, $CH_3$—N<),
2.45 to 2.80 (4H, m, 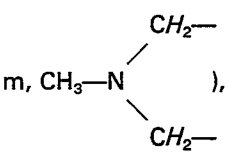 ), 3.40 to 3.80 (4H, m, 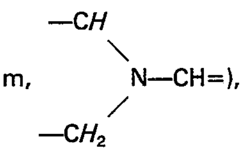 ),
4.35 (2H, s, —$CH_2$COO—), 7.45 (1H, s, —CH=).
EA: shown in Table 3.

Example 30

[3-Carboxymethyl-5-(2-biphenylyl)aminomethylidenerhodanine (Compound No. 28)]

The same procedures as in Example 19 except that o-phenylaniline was used instead of allylamine and the reaction was conducted at a room temperature for 4 to 20 hours to give 1.36 g of 3-carboxymethyl-5-(2-biphenylyl)aminomethylidenerhodanine in the form of yellow crystal (yield: 71.5%).

The characteristic properties of the product are as follows:
MP: 248° to 250°C (decomposition)
IR ($cm^{-1}$): 3300 to 2850 (NH, CH), 2650 to 2350 (COOH), 1735 (ring C=O), 1660 (COOH)
MS (m/e): 370 ($M^+$)
NMR (ppm): 4.40 (2H, s, —$CH_2$COO—), 6.95 to 7.40 (9H, m, aromatic proton), 7.55 (1H, d, —CH=), 9.80 (1H, d, —NH—)
EA: shown in Table 3.

Example 31

[3-Carboxymethyl-5-m-methoxyphenylaminomethylidenerhodanine sodium salt (sodium salt of Compound No. 20)]

129.6 milligrams (0.0004 mole) of 3-carboxymethyl-5-m-methoxyphenylaminomethylidenerhodanine prepared in Example 22 was dissolved in 4 ml of 0.1N sodium hydroxide and the resulting solution was freeze-dried to quantitavely give the desired compound in the form of yellow powder.

MP: 268° to 270°C (decomposition)

Sodium salts of Compound Nos. 14 to 15, Nos. 17 to 19 and Nos. 21 to 28 were obtained in the same manner as described above.

Example 32

[3-Carboxymethyl-5-m-methoxyphenylaminomethylidenerhodanine potassium salt (potassium salt of Compound No. 20)]

129.6 milligrams (0.0004 mole) of 3-carboxymethyl-5-m-methoxyphenylaminomethylidene rhodanine

was dissolved in 4 ml of 0.1N potassium hydroxide and the resulting solution was freeze-dried to quantitatively give the desired compound in the form of red powder.

MP: 280° to 282°C (decomposition)

Potassium salts of Compound Nos. 14 to 15, Nos. 17 to 19 and Nos. 21 to 28 were obtained in the same manner as described above.

Table 3

| Compound No. | Molecular formula | Molecular weight | Calcd. (%) | | | Found (%) | | |
|---|---|---|---|---|---|---|---|---|
| | | | C | H | N | C | H | N |
| 14 | $C_7 H_8 N_2 O_3 S_2$ | 232.282 | 36.20 | 3.47 | 12.6 | 36.39 | 3.72 | 12.33 |
| 15 | $C_8 H_{10} N_2 O_3 S_2$ | 246.309 | 39.01 | 4.09 | 11.37 | 38.96 | 4.28 | 11.29 |
| 16 | $C_8 H_{10} N_2 O_4 S_2$ $HOCH_2 CH_2 NH_2$ | 323.393 | 37.14 | 5.30 | 13.00 | 36.92 | 5.46 | 13.26 |
| 17 | $C_9 H_{10} N_2 O_3 S_2$ | 258.320 | 41.85 | 3.90 | 10.84 | 41.78 | 3.81 | 11.03 |
| 18 | $C_{10} H_{14} N_2 O_3 S_2$ | 274.364 | 43.78 | 5.14 | 10.21 | 43.57 | 4.97 | 10.36 |
| 19 | $C_{12} H_{18} N_2 O_3 S_2$ | 302.418 | 47.66 | 6.00 | 9.26 | 47.52 | 5.79 | 9.42 |
| 20 | $C_{13} H_{12} N_2 O_4 S_2$ | 324.380 | 48.14 | 3.73 | 8.64 | 48.35 | 3.72 | 8.50 |
| 21 | $C_{13} H_{12} N_2 O_4 S_2$ | 324.380 | 48.14 | 3.73 | 8.64 | 47.99 | 3.73 | 8.44 |
| 22 | $C_{12} H_9 N_2 O_3 S_2 Cl$ | 328.799 | 43.84 | 2.76 | 8.52 | 43.63 | 2.70 | 8.26 |
| 23 | $C_{12} H_9 N_2 O_3 S_2 Br$ | 373.255 | 38.62 | 2.43 | 7.51 | 38.57 | 2.38 | 7.67 |
| 24 | $C_{13} H_{12} N_2 O_3 S_2$ | 308.381 | 50.63 | 3.92 | 9.08 | 50.64 | 3.98 | 8.91 |
| 25 | $C_{10} H_{12} N_2 O_4 S_2$ | 288.347 | 41.66 | 4.19 | 9.72 | 41.91 | 4.34 | 9.63 |
| 26 | $C_{11} H_{15} N_3 O_3 S_2$ | 301.389 | 43.84 | 5.02 | 13.94 | 43.63 | 5.14 | 14.03 |
| 27 | $C_{12} H_{17} N_3 O_3 S_2$ | 315.416 | 45.70 | 5.43 | 13.32 | 45.55 | 5.15 | 13.12 |
| 28 | $C_{18} H_{14} N_2 O_3 S_2$ | 370.453 | 58.36 | 3.81 | 7.56 | 58.29 | 3.77 | 7.80 |

Example 33

A mixture of 10 parts by weight of 3-carboxymethyl-5-(2-butenylidene)rhodanine (Compound No. 7), 30 parts of lactose, 45 parts by weight of corn starch, 15 parts by weight of a microcrystalline cellulose (commercially available under the registered trade mark "Avicel" made by Asahi Chemical Industry Co., Ltd.), 3 parts by weight of methyl cellulose and 2 parts by weight of magnesium stearate was thoroughly blended and then screened through a 50 mesh screen. The resulting powder was tabletted by an automatic tabletting machine to give tablets containing 20 mg of the essential active ingredient per one tablet.

Example 34

The same procedures as in Example 33 except that 3-carboxymethyl-5-m-methoxyphenylaminomethylidenerhodanine (Compound No. 20) was used as the essential active ingredient were repeated to give tablets.

Example 35

A mixture of 10 parts by weight of 3-carboxymethyl-5-(2-hexenylidene)rhodanine (Compound No. 10), 55 parts by weight of lactose, 30 parts by weight of corn starch, 8 parts by weight of Avicel and 2 parts by weight of magnesium stearate was thoroughly blended. The mixture was then filled in capsules made of gelatin to give capsules containing 20 mg of the essential active ingredient per one capsule.

Example 36

The same procedures as in Example 35 except that 3-carboxymethyl-5-m-chlorophenylaminomethylidenerhodanine (Compound No. 22) was used as the essential active ingredient were repeated to give capsules.

Example 37

The tablets obtained in Example 33 were pulverized and the resulting powder was screened through a 50 mesh screen and a 100 mesh screen to give granules having a particle size of 5 to 100 meshes and containing 50 mg of the essential active ingredient per 1 g of granules.

Example 38

The same mixture used in Example 35 was finely pulverized and then screened through a 100 mesh screen to give a powder having an average particle size of 100 meshes and containing 50 mg of the essential active ingredient per 1 g of powder.

Example 39

[Platelet aggregation inhibiting activity]

The platelet aggregation inhibiting activity was examined with the rhodanine derivatives (I) of the present invention.

*Test method*

Blood samples were collected from the auricular blood vessel of albino rabbits (white local breed), and washed platelets were prepared therefrom by the method of Baenziger et al. [N. L. Baenziger and P. W. Majerus, Methods in Enzymology, *31*, 149 to 155 (1974)]. The platelets were suspended in a 15 mM Tris-hydrochloric acid buffer in a final concentration of $6 \times 10^8$ cells/ml (Tris: tris(hydroxymethyl)aminomethane). Each test compound was added thereto and incubation was carried out at 37°C for 2 minutes. Then, the platelets were stimulated by addition of thrombin (final concentration 0.2 unit/ml; made by Mochida Pharmaceutical Co.) and the aggregation inhibiting activity was estimated by observation of the aggregation reaction using an aggregometer (made by Briston Co.).

The results obtained are shown in Table 4 and Table 5 in terms of $IC_{50}$ (50% inhibition concentration in M).

Table 4

| Compound No. | $IC_{50}$ (M) |
|---|---|
| 1 | >3 X $10^{-4}$ |
| 2 | >3 X $10^{-4}$ |
| 3 | >3 X $10^{-4}$ |
| 4 | >3 X $10^{-4}$ |
| 5 | >3 X $10^{-4}$ |
| 6 | 3 X $10^{-5}$ |
| 7 | 2 X $10^{-5}$ |
| 8 | 2 X $10^{-5}$ |
| 9 | >4 X $10^{-4}$ |
| 10 | 3 X $10^{-5}$ |
| 11 | 2 X $10^{-5}$ |
| 12 | >3 X $10^{-4}$ |
| 13 | 4 X $10^{-5}$ |
| Dipyridamole | 2 X $10^{-4}$ |

Table 5

| Compound No. | $IC_{50}$ (M) |
|---|---|
| 20 | 3.1 X $10^{-4}$ |
| 21 | 3.1 X $10^{-4}$ |
| 22 | 3.0 X $10^{-4}$ |
| 23 | 3.0 X $10^{-4}$ |
| 24 | 3.2 X $10^{-4}$ |
| 28 | 4.1 X $10^{-4}$ |
| Dipyridamole | 2.0 X $10^{-4}$ |

As shown in Table 4, Compound Nos. 6, 7, 8, 10, 11 and 13 which belong to the rhodanine derivative (Ia) inhibited the thrombin-induced aggregation of washed rabbit platelets by 50% at a concentration of $2 \times 10^{-5}$ to $4 \times 10^{-5}$ M. With other compounds belonging to the rhodanine derivative (Ia), too, platelet aggregation inhibiting activity was observed, though it was weaker than that of the above six compounds. The activity of the above six compounds was about 10 times that of Dipyridamole, a platelet aggregation inhibitor in wide use.

As shown in Table 5, Compound Nos. 20, 21, 22, 23 and 24 which belong to the rhodanine derivative (Ib) inhibited the thrombin-induced aggregation of washed rabbit platelets by 50% at a concentration of $3 \times 10^{-4}$ M. With other compounds belonging to the rhodanine derivative (Ib), too, platelet aggregation inhibiting activity was observed, though it was weaker than that of the above five compounds. The above five compounds were almost comparable in said activity to Dipyridamole.

Example 40

[Aldose reductase inhibiting activity]

The aldose reductase inhibiting activity was examined with respect to the rhodanine derivative (I) of the present invention.

*Test Method*

The test was carried out according to the method of Hayman et al. [S. Hayman and J. H. Kinoshita, J. Biol. Chem., *240*, 877 to 882 (1965)].

Thus, Wistar strain male rats were sacrificed by decapitation, the lenses were excised and homogenized with a 0.1 M phosphate buffer [pH 6.8; containing 1 mM of mercaptoethanol and 1 mM of nicotinamide-adenine dinucleotide phosphate (NADP)]. The homogenate was then centrifuged at 10,000 g for 15 minutes and the supernatant was used as the crude enzyme solution.

Separately, a 0.1 M phosphate buffer (pH 6.2) containing 0.104 mM of NADPH (reduced form of NADP) and 10 mM of DL-glyceraldehyde was prepared. To this buffer solution, there was added 15 μl of each of solutions of each test compound in varied concentrations, followed by addition of 25 μl of the crude enzyme solution prepared in advance, to thereby initiate the reaction. The decrease in absorbance at 340 nm was measured using a high-sensitivity self-registering spectrophotometer (Model SM—401 made by Union Giken Kabushiki Kaisha).

The results obtained are shown in Table 6 and Table 7 in term of $IC_{50}$ (50% inhibition concentration in M).

Table 6

| Compound No. | $IC_{50}$ (M) |
|---|---|
| 1 | $2 \times 10^{-7}$ |
| 2 | $1.4 \times 10^{-7}$ |
| 3 | $1 \times 10^{-7}$ |
| 4 | $3 \times 10^{-7}$ |
| 5 | $1.3 \times 10^{-7}$ |
| 6 | $7.5 \times 10^{-7}$ |
| 7 | $7 \times 10^{-8}$ |
| 8 | $1.3 \times 10^{-7}$ |
| 9 | $1 \times 10^{-7}$ |
| 10 | $4 \times 10^{-8}$ |
| 11 | $4.6 \times 10^{-8}$ |
| 12 | 7 to $8 \times 10^{-8}$ |
| 13 | $6.5 \times 10^{-7}$ |

Table 7

| Compound No. | $IC_{50}$ (M) |
|---|---|
| 14 | $1.3 \times 10^{-6}$ |
| 15 | $6.5 \times 10^{-7}$ |
| 16 | $7.0 \times 10^{-7}$ |
| 17 | $3.0 \times 10^{-7}$ |
| 18 | $6.0 \times 10^{-7}$ |
| 19 | $2.8 \times 10^{-7}$ |
| 20 | $2.6 \times 10^{-8}$ |
| 21 | $5.0 \times 10^{-8}$ |
| 22 | $2.3 \times 10^{-8}$ |
| 23 | $5.0 \times 10^{-8}$ |
| 24 | $3.6 \times 10^{-8}$ |
| 25 | $3.4 \times 10^{-7}$ |
| 26 | $2.0 \times 10^{-7}$ |
| 27 | $1.3 \times 10^{-6}$ |
| 28 | $1.5 \times 10^{-7}$ |
| Sorvinyl (Note) | $2.0 \times 10^{-7}$ |

Note: the registered trademark of S-6-fluoro-spiro(chroman-4,4'-imidazolidine)-2',5'-dione having the following formula:

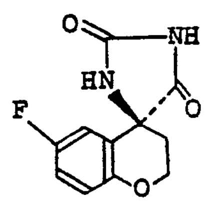

(made by Pfizer Inc.)

In the laboratory test for inhibitory activity against aldose reductase obtained from the rat eye lens, all the compounds listed in Tables 6 and 7 showed 50% inhibition within the concentration range of $10^{-8}$ to $10^{-6}$ M.

Example 41

[Acute toxicity]

The acute toxicity in mice was examined with the rhodanine derivative (I) of the present invention.

*Test Method*

To groups of 4 male ddY strain mice (5 weeks of age) were orally administered by gavage each test compound suspended in a 10% gum arabic and the mice were observed for death or survival for 2 weeks.

The results obtained with respect to Compound Nos. 7, 10, 11, 12 and 13 belonging to the rhodanine derivative (Ia) are shown in Table 8.

Table 8

| Oral dose (g/kg body weight) | Rate of death (%) |
|---|---|
| 1.0 | 0 |
| 2.5 | 0 |
| 3.5 | 0 |
| 5.0 | 100 |

As shown in Table 8, with respect to Compound Nos. 7, 10, 11, 12 and 13, $LD_{50}$ value (oral) was very high, namely in the range of 3.5 to 5.0 g/kg body weight.

The results obtained with respect to Compound Nos. 20, 22, 24 and 28 belonging to the rhodanine derivative (Ib) are shown in Table 9.

Table 9

| Oral dose (g/kg body weight) | Rate of death (%) |
|---|---|
| 1.0 | 0 |
| 2.0 | 0 |
| 4.0 | 0 |

As shown in Table 9, with respect to Compound Nos. 20, 22, 24 and 28, no deaths were noted up to a dose of 4 g/kg body weight, and therefore the $LD_{50}$ value (oral) for each compound was greater than 4 g/kg body weight.

It was thus demonstrated that the rhodanine derivatives (I) of the present invention are of very low toxicity.

As above results indicate, the rhodanine derivatives (I) of the present invention can be used as novel and highly safe therapeutic agents for diabetic complications which have not only potent platelet aggregation inhibiting activity but also aldose reductase inhibiting activity much more potent as compared with the known compounds having aldose reductase inhibiting activity.

In addition to the ingredients used in the Examples, other ingredients can be used in the Examples as set forth in the specification to obtain substantially the same results.

**Claims**

1. A rhodanine derivative having the following general formula (I):

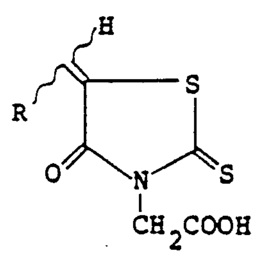

(I)

wherein R is an acyclic alkyl group having 2 to 10 carbon atoms which may be substituted by a halogen atom; an acyclic alkenyl group or polyenyl group having 1 to 4 olefinic double bonds having 2 to 19 carbon atoms which may be substituted by a halogen atom; a mono- or di-alkylamino group having 1 to 6 carbon atoms which may be substituted by a hydroxyl group; an alkenylamino group having 3 to 6 carbon atoms; a phenylamino group, the benzene ring of which may be substituted by a $C_1$—$C_4$ alkyl group, a $C_1$—$C_4$ alkoxyl group, phenyl group or a halogen atom; or a cyclic amino group which may contain in the ring an oxygen atom or a nitrogen atom to which a $C_1$—$C_3$ alkyl group is attached; or a nontoxic salt thereof.

2. The rhodanine derivative of Claim 1, wherein R is ethyl, 3-chloropropyl, t-butyl, n-decyl, n-pentyl, vinyl, 1-propenyl, 3-chloro-1-propenyl, 2-methyl-1-propenyl, 1-pentenyl, 1,3-pentadienyl, 2,6-dimethyl-1,5-heptadienyl or 2,6,10,14-tetramethyl-1,5,9,13-pentadecatetraenyl group.

3. The rhodanine derivative of Claim 1, wherein R is methylamino, dimethylamino, 2-hydroxyethylamino, allylamino, isobutylamino, hexylamino, 2-biphenylamino, 3-methoxyphenylamino, 2-methoxyphenylamino, 3-chlorophenylamino, 4-bromophenylamino, m-tolylamino, morpholino, piperidino, 4-methylpiperazin-1-yl or 4-methylhomopiperazin-1-yl group.

4. A process for preparing a rhodanine derivative having the following general formula (Ia):

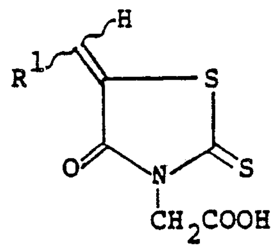

(Ia)

wherein $R^1$ is an acyclic alkyl group having 2 to 10 carbon atoms which may be substituted by a halogen atom or an acyclic alkenyl group or polyenyl group having 1 to 4 olefinic double bonds having 2 to 19 carbon atoms which may be substituted by a halogen atom, or its nontoxic salt, which comprises reacting 3-carboxymethylrhodanine having the following formula (II):

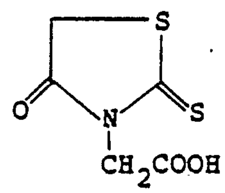

(II)

or a salt thereof with an aldehyde compound having the following general formula (III):

$$R^1\text{—CHO} \quad \text{(III)}$$

wherein $R^1$ is an acyclic alkyl group having 1 to 9 carbon atoms which may be substituted by a halogen atom or an acyclic alkenyl group having 2 to 18 carbon atoms which may be substituted by a halogen atom.

5. The process of Claim 4, wherein $R^1$ is as in claim 2.

6. A process for preparing a rhodanine derivative having the following general formula (Ib):

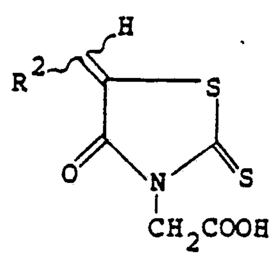

(Ib)

wherein $R^2$ is a mono- or di-alkylamino group having 1 to 6 carbon atoms which may be substituted by a hydroxyl group; an alkenylamino group having 3 to 6 carbon atoms; a phenylamino group, the benzene ring of which may be substituted by a $C_1$—$C_4$ alkyl group, a $C_1$—$C_4$ alkoxyl group, phenyl group or a halogen atom; or a cyclic amino group which may contain in the ring an oxygen atom or a nitrogen atom to which a $C_1$—$C_3$ alkyl group is attached, or its nontoxic salt, which comprises reacting a 3-carboxymethylrhodanine derivative having the following general formula (IV):

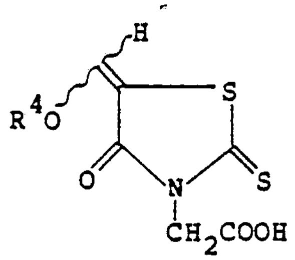

(IV)

wherein $R^4$ is an $C_1$—$C_4$ alkyl group, or a salt thereof with an amine compound having the following general formula (V):

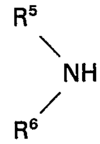

(V)

wherein $R^5$ is hydrogen atom or a $C_1$—$C_4$ alkyl group, and $R^6$ is an alkyl group having 1 to 6 carbon atoms which may be substituted by a hydroxyl group; an alkenyl group having 3 to 6 carbon atoms; phenyl group, the benzene ring of which may be substituted by a $C_1$—$C_4$ alkyl group, a $C_1$—$C_4$ alkoxyl group, phenyl group or a halogen atom; or $R^5$ and $R^6$ may be combined together with the nitrogen atom to which $R^5$ and $R^6$ attach to form a ring which may contain in the ring an oxygen atom or a nitrogen atom to which a $C_1$—$C_3$ alkyl group is attached.

7. The process of Claim 6, wherein $R^2$ is as in claim 3.

8. A pharmaceutical composition as a therapeutic agent for diabetic complications which comprises as an essential active ingredient an effective amount of a rhodanine derivative having the following general formula (I):

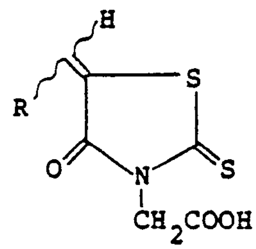

(I)

wherein R is as in claim 1.

9. The pharmaceutical composition of Claim 8, wherein R is as in claim 2.

10. The pharmaceutical composition of Claim 8, wherein R is as in claim 3.

**Patentansprüche**

1. Rhodaninderivat mit der folgenden allgemeinen Formel (I):

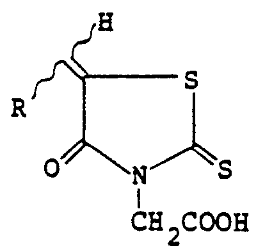

(I)

worin R eine acyclische Alkylgruppe mit 2 bis 10 Kohlenstoffatomen ist, die substituiert sein kann mit einem Halogenatom; eine acyclische Alkenylgruppe oder Polyenylgruppe mit 1 bis 4 olefinischen Doppelbindungen mit 2 bis 19 Kohlenstoffatomen, die substituiert sein können mit einem Halogenatom; eine Mono- oder Dialkylaminogruppe mit 1 bis 6 Kohlenstoffatomen, die substituiert sein können mit einer Hydroxylgruppe; eine Alkenylaminogruppe mit 3 bis 6 Kohlenstoffatomen; eine Phenylaminogruppe, deren Benzolring substituiert sein kann mit einer $C_1$—$C_4$-Alkylgruppe, einer $C_1$—$C_4$-Alkoxygruppe, Phenylgruppe oder einem Halogenatom; oder eine cyclische Aminogruppe, die im Ring ein Sauerstoffatom oder ein Stickstoffatom enthalten kann, an die eine $C_1$—$C_3$-Alkylgruppe gebunden ist; oder ein nicht-toxisches Salz davon.

2. Rhodaninderivat nach Anspruch 1, worin R Ethyl-, 3-Chlorpropyl-, t-Butyl, n-Decyl-, n-Pentyl-, Vinyl-, 1-Propenyl-, 3-Chlor-1-propenyl-, 2-Methyl-1-propenyl-, 1-Pentenyl-, 1,3-Pentadienyl-, 2,6-Dimethyl-1,5-heptadienyl- oder 2,6,10,14-Tetramethyl-1,5,9,13-pentadecatetraenylgruppe ist.

3. Rhodaninderivat nach Anspruch 1, worin R Methylamino-, Dimethylamino-, 2-Hydroxyethylamino-, Allylamino-, Isobutylamino-, Hexylamino-, 2-Biphenylylamino-, 3-Methoxyphenylamino-, 2-Methoxyphenylamino-, 3-Chlor-phenylamino-, 4-Bromphenylamino-, m-Tolylamino-, Morpholino-, Piperidino-, 4-Methylpiperazin-1-yl- oder 4-Methylhomopiperazin-4-yl-gruppe ist.

4. Verfahren zur Herstellung eines Rhodaninderivates mit der folgenden allgemeinen Formel (Ia):

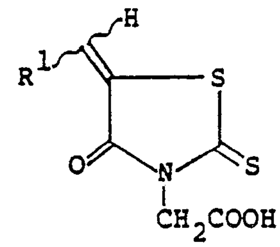

(Ia)

worin $R^1$ eine acyclische Alkylgruppe mit 2 bis 10 Kohlenstoffatomen ist, die substituiert sein kann mit einem Halogenatom oder eine acyclische Alkenylgruppe oder Polyenylgruppe mit 1 bis 4 olefinischen Doppelbindungen mit 2 bis 19 Kohlenstoffatomen, die substituiert sein können mit einem Halogenatom, oder deren nicht-toxisches Salz, welches umfaßt Umsetzen von 3-Carboxymethylrhodanin mit der folgenden Formel (II):

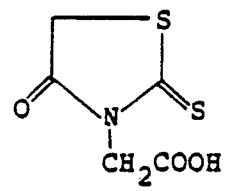

(II)

oder eines Salzes davon mit einer Aldehydverbindung mit der folgenden allgemeinen Formel (III):

$$R^1—CHO \qquad (III)$$

worin $R^1$ eine acyclische Alkylgruppe mit 1 bis 9 Kohlenstoffatomen ist, die substituiert sein kann mit einem Halogenatom oder eine acyclische Alkenylgruppe mit 2 bis 18 Kohlenstoffatomen, die substituiert sein kann mit einem Halogenatom.

5. Verfahren nach Anspruch 4, worin $R^1$ wie in Anspruch 2 ist.

6. Verfahren zur Herstellung eines Rhodaninderivates mit der folgenden allgemeinen Formel (Ib):

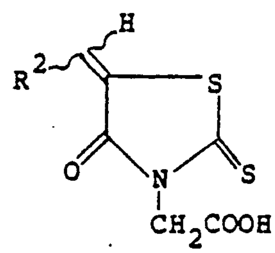

(Ib)

worin $R^2$ eine Mono- oder Dialkylaminogruppe mit 1 bis 6 Kohlenstoffatomen ist, die mit einer Hydroxylgruppe substituiert sein können; eine Alkenylaminogruppe mit 3 bis 6 Kohlenstoffatomen; eine Phenylaminogruppe, deren Benzolring substituiert sein kann mit einer $C_1—C_4$-Alkylgruppe, einer $C_1—C_4$-Alkoxylgruppe, Phenylgruppe oder einem Halogenatom; oder eine cyclische Aminogruppe, die im Ring ein Sauerstoffatom oder ein Stickstoffatom enthalten kann, an die eine $C_1—C_3$-Alkylgruppe gebunden ist, oder deren nicht-toxischen Salzes welches umfaßt Umsetzen eines 3-Carboxymethylrhodaninderivates mit der folgenden allgemeinen Formel (IV):

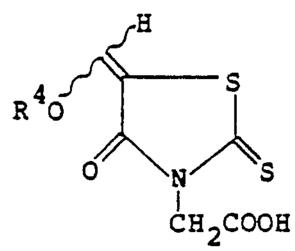

(IV)

worin $R^4$ eine $C_1—C_4$-Alkylgruppe ist, oder eines Salzes davon mit einer Aminverbindung mit der folgenden allgemeinen Formel (V):

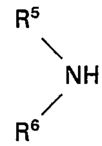

(V)

worin $R^5$ Wasserstoffatom oder eine $C_1$—$C_4$-Alkylgruppe ist, und $R^6$ eine Alkylgruppe mit 1 bis 6 Kohlenstoffatomen ist, die substituiert sein kann mit einer Hydroxylgruppe; eine Alkenylgruppe mit 3 bis 6 Kohlenstoffatomen; Phenylgruppe, deren Benzolring substituiert sein kann mit einer $C_1$—$C_4$-Alkylgruppe, $C_1$—$C_4$-Alkoxylgruppe, Phenylgruppe oder einem Halogenatom; oder $R^5$ und $R^6$ können kombiniert werden mit dem Stickstoffatom, an das $R^5$ und $R^6$ gebunden sind, um einen Ring zu bilden, der im Ring ein Sauerstoffatom oder ein Stickstoffatom enthalten kann, an die eine $C_1$—$C_3$-Alkylgruppe gebunden ist.

7. Verfahren nach Anspruch 6, worin $R^2$ wie in Anspruch 3 ist.

8. Pharmazeutische Zusammensetzung als ein therapeutisches Mittel bei Diabetes-Komplikationen, welches umfaßt als einen wesentlichen aktiven Bestandteil eine wirksame Menge eines Rhodaninderivates mit der folgenden allgemeinen Formel (I):

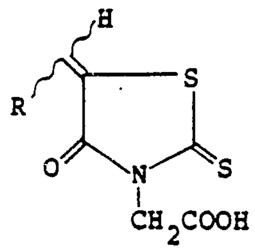

(I)

worin R wie in Anspruch 1 ist.

9. Pharmazeutische Zusammensetzung nach Anspruch 8, worin R wie in Anspruch 2 ist.

10. Pharmazeutische Zusammensetzung nach Anspruch 8, worin R wie in Anspruch 3 ist.

**Revendications**

1. Dérivé de rhodanine de la formule générale suivante (I):

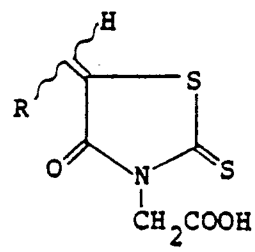

(I)

où R est un radical alcoyle acyclique de 2 à 10 atomes de carbone qui peut être substitué par un atome d'halogène; un radical alcényle acyclique ou radical polyényle comptant 1 à 4 doubles liaisons oléfiniques de 2 à 19 atomes de carbone qui peut être substitué par un atome d'halogène; un radical mono- ou di-alcoylamino de 1 à 6 atomes de carbone qui peut être substitué par un radical hydroxyle; un radical alcénylamino de 3 à 6 atomes de carbone; un radical phénylamino dont le cycle benzénique peut être substitué par un radical alcoyle en $C_1$—$C_4$, un radical alcoxy en $C_1$—$C_4$, un radical phényle ou un atome d'halogène; ou un radical amino cyclique qui peut contenir dans le cycle un atome d'oxygène ou un atome d'azote auquel un radical alcoyle en $C_1$—$C_3$ est fixé; ou un sel non toxique de celui ci.

2. Dérivé de rhodanine suivant la revendication 1, dans lequel R est un radical éthyle, 3-chloropropyle, t-butyle, n-décyle, n-pentyle, vinyle, 1-propényle, 3-chloro-1-propényle, 2-méthyl-1-propényle, 1-pentényle, 1,3-pentadiényle, 2,6-diméthyl-1,5-heptadiényle ou 2,6,10,14-tétraméthyl-1,5,9,13-pentadécatétraényle.

3. Dérivé de rhodanine suivant la revendication 1, dans lequel R est un radical méthylamino, diméthylamino, 2-hydroxyéthylamino, allylamino, isobutylamino, hexylamino, 2-biphénylylamino, 3-méthoxyphénylamino, 2-méthoxyphénylamino, 3-chlorophénylamino, 4-bromophénylamino, m-tolylamino, morpholino, pipéridino, 4-méthylpipérazin-1-yle ou 4-méthylhomopipérazin-1-yle.

4. Procédé de préparation d'un dérivé de rhodanine de la formule générale suivante (Ia):

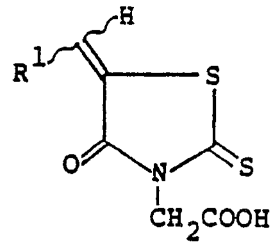

(Ia)

où $R^1$ est un radical alcoyle acyclique de 2 à 10 atomes de carbone qui peut être substitué par un atome d'halogène; ou un radical alcényle acyclique ou radical polyényle comptant 1 à 4 doubles liaisons oléfiniques de 2 à 19 atomes de carbone qui peut être substitué par un atome d'halogène, ou de son sel non toxique qui comprend la réaction de la 3-carboxyméthylrhodanine de la formule suivante (II):

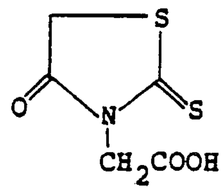

(II)

ou d'un sel de celle-ci avec un composé aldéhydique de la formule générale suivante (III):

$$R^1—CHO \qquad (III)$$

où $R^1$ est un radical alcoyle acyclique de 1 à 9 atomes de carbone qui peut être substitué par un atome d'halogène; ou un radical alcényle acyclique ou polyényle comptant 1 à 4 doubles liaisons oléfiniques de 2 à 18 atomes de carbone qui peut être substitué par un atome d'halogène.

5. Procédé suivant la revendication 4, dans lequel $R^1$ est tel que défini dans la revendication 2.

6. Procédé de préparation d'un dérivé de rhodanine de la formule générale suivante (Ib):

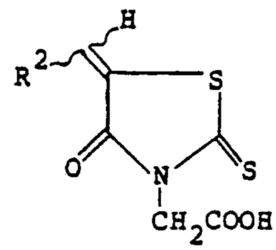

(Ib)

où $R^2$ est un radical mono- ou di-alcoylamino de 1 à 6 atomes de carbone qui peut être substitué par un radical hydroxyle; un radical alcénylamino de 3 à 6 atomes de carbone; un radical phénylamino dont le cycle benzénique peut être substitué par un radical alcoyle en $C_1—C_4$, un radical alcoxy en $C_1—C_4$, un radical phényle ou un atome d'halogène; ou un radical amino cyclique qui peut contenir dans le cycle un atome d'oxygène ou un atome d'azote auquel un radical alcoyle en $C_1—C_3$ est fixé, ou de son sel non toxique, qui comprend la réaction d'un dérivé 3-carboxyméthylrhodanine de la formule générale suivante (IV):

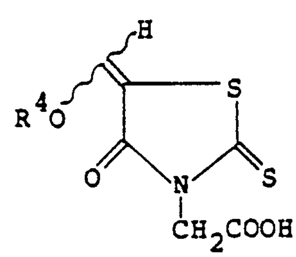

(IV)

où $R^4$ est un radical alcoyle en $C_1—C_4$, ou d'un sel de celui-ci, avec une amine de la formule générale suivante (V):

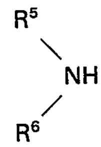

(V)

où $R^5$ est un atome d'hydrogène ou un radical alcoyle en $C_1$—$C_4$, et $R^6$ est un radical alcoyle de 1 à 6 atomes de carbone qui peut être substitué par un radical hydroxyle; un radical alcényle de 3 à 6 atomes de carbone; un radical phényle dont le cycle benzénique peut être substitué par un radical alcoyle en $C_1$—$C_4$, un radical alcoxy en $C_1$—$C_4$, un radical phényle ou un atome d'halogène; ou bien $R^5$ et $R^6$ peuvent être combinés conjointement avec l'atome d'azote auquel $R^5$ et $R^6$ sont fixés pour former un cycle qui peut contenir dans le cycle un atome d'oxygène ou un atome d'azote auquel un radical alcoyle en $C_1$—$C_3$ est fixé.

7. Procédé suivant la revendication 6, dans lequel $R^2$ est tel que défini dans la revendication 3.

8. Composition pharmaceutique comme agent thérapeutique pour les complications diabétiques, qui comprend comme constituant actif essentiel une quantité efficace d'un dérivé de rhodanine de la formule générale suivante (I):

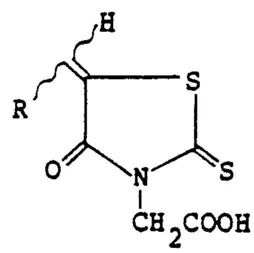

(I)

où R est tel que défini dans la revendication 1.

9. Composition pharmaceutique suivant la revendication 8, dans laquelle R est tel que défini dans la revendication 2.

10. Composition pharmaceutique suivant la revendication 8, dans laquelle R est tel que défini dans la revendication 3.